# FASCICULE DE BREVET EUROPEEN

(11) **EP 2 079 528 B1**
(45) Date de publication et mention de la délivrance du brevet: **21.07.2010**
(21) Numéro de dépôt: 07823849.0
(22) Date de dépôt: 25.09.2007
(51) Int. Cl.: A61Q 5/02, A61Q 13/00, A61Q 15/00, A61Q 19/10, A61K 8/49, A23L 1/226, C11D 3/50

(54) **UTILISATION EN PARFUMERIE ET EN AROMATIQUE ET PROCEDE DE PREPARATION DE LA 5,5-DIMETHYL-3-ETHYL-3, 4-DIHYDRO-FURAN-2-ONE**
VERWENDUNG IN DER PARFÜMERIE UND ALS AROMASTOFF UND VERFAHREN ZUR HERSTELLUNG VON 5,5-DIMETHYL-3-ETHYL-3,4-DIHYDROFURAN-2-ON
USE IN PERFUMERY AND FLAVOURING AND PROCESS FOR THE PREPARATION OF 5,5-DIMETHYL-3-ETHYL-3,4-DIHYDROFURAN-2-ONE

(30) Priorité: 03.10.2006 FR 0608661
(43) Date de publication de la demande: 22.07.2009
(73) Titulaire: V. Mane Fils, 06620 Bar sur Loup (FR)
(72) Inventeur: MANE, Jean, 06130 Grasse (FR); CLINET, Jean-Claude, 06270 Villeneuve-Loubet (FR); CLINET, Isabel, 06270 Villeneuve-Loubet (FR); COULOMBEL, Lydie, 06370 Mouans-Sartoux (FR); MARIN, Christophe, 06200 Nice (FR)
(74) Mandataire: Cabinet Plasseraud
(86) Numéro de dépôt international: PCT/FR2007/052004
(87) Numéro de publication internationale: WO 2008/040897

(56) Documents cités:
- FUJITA, TSUTOMU ET AL: "A convenient one step synthesis of 2,4-dialkyl-2-hydroxyalkyl-.gamma.- butyrolactone using lithium naphthalenide" JOURNAL OF APPLIED CHEMISTRY & BIOTECHNOLOGY, CODEN: JACBBD; ISSN: 0375-9210, vol. 27, no. 10, 1977, pages 539-542, XP009084137 cité dans la demande
- KIRMSE, WOLFGANG ET AL: "Stereochemistry of aliphatic carbocations. 9. Substituent effects of 1,2-hydride shifts" CHEMISCHE BERICHTE , CODEN: CHBEAM; ISSN: 0009-2940, vol. 108, no. 6, 1975, pages 1839-1854, XP009084154 cité dans la demande

## Description

La présente invention se rapporte à l'utilisation de la 5,5-diméthyl-3-éthyl-3,4-dihydro-furan-2-one, appelée « Noxolide » par la Demanderesse, comme agent odorant, en parfumerie ou aromatique alimentaire.

L'invention concerne également un nouveau procédé de préparation de la 5,5-diméthyl-3-éthyl-3,4-dihydro-furan-2-one.

Les termes « fragrance », « fragrant », « odorant » sont utilisés ici de manière interchangeable pour tout composé organoleptique stimulant de façon plaisante l'odorat.

Les termes « arôme » et « aromatique » sont utilisés ici de manière interchangeable pour tout composé organoleptique stimulant le goût.

Le terme « composé organoleptique » est utilisé ici pour tout composé stimulant les sens de l'odorat et du goût, et étant donc perçu comme possédant une odeur et/ou un goût caractéristique.

Par le terme « masquer » ou « masquant » on entend réduire ou éliminer la perception d'une mauvaise odeur ou d'un mauvais goût générée par une ou plusieurs molécules entrant dans la composition d'un produit.

Les molécules odorantes appartiennent à des classes chimiques diverses. On peut notamment citer les lactones largement répandues à l'état naturel et constituant une famille de molécules odorantes très importante en parfumerie. Les lactones sont connues pour leurs notes fruitées, comme par exemple la lactone γ-Ncnanoïque (fragrance de noix de coco), décrite dans Bunce, R.A. ; Reeves, H.D ; J.Chem.Ed. 1990, 67, 69-70. D'autres fragrances bien connues des lactones sont décrites avec des termes comme herbe, foin, épicé, boisé, amande, lait, fruits sucrés comme la pêche ou l'abricot.

Les lactones sont présentes sous forme de traces dans les fruits, les produits lactés et les boissons alcoolisées mais contribuent substantiellement à leurs arômes (Duffosé L. et al; Sciences des Aliments 1994, 14, 17-50). Le faible seuil de détection olfactive des lactones est compensé par une utilisation en forte quantité de celles-ci dans le domaine des parfums et des arômes (Zope D.D. et al; Fafai Journal 2004, 43-54).

Certaines lactones apportent la note principale d'un arôme donné ; celle-ci est souvent agréable et persistante. La Demanderesse a mis en évidence au cours de ses recherches que la 5,5-diméthyl-3-éthyl-3,4-dihydro-furan-2-one, appartenant à la famille des γ-lactones tri-substituées, désignée sous le nom de « Noxolide », présente de telles caractéristiques.

A la connaissance de la Demanderesse, la 5,5-diméthyl-3-éthyl-3,4-dihydro-furan-2-one a été reportée deux fois dans la littérature. Premièrement lors d'une étude sur les effets de substituant dans les réactions de transfert 1,2-hydrures (Kirmse W. et al; Chem. Ber. 1975, 108, 1839 & M. de Campos M. et al; Arch. Pharm., (Weinheim) 1965, 298, 92). Plus récemment, la 5,5-diméthyl-3-cthyl-3,4-dihydro-furan-2-one a été décrite comme l'un des produits de la réaction de l'acide butanoïque avec le complexe organométallique « naphtalène/lithium » suivi d'une condensation avec l'oxyde d'isobutène (Fujita et al; J. Appl. Chem. Biotechnol., 1977, 27, 539).

Cependant, aucun des documents de l'art antérieur ne décrit ni ne suggère que la 5,5-diméthyl-3-éthyl-3,4-dihydro-furan-2-one ait des propriétés organoleptiques, et *a fortiori,* il n'est ni décrit ni suggéré l'utilisation qui pourrait être faite de cette molécule en tant que composé odorant, notamment fragrance ou en tant qu'arôme, ou encore en tant qu'agent masquant d'une odeur ou d'une flaveur.

Par ailleurs, les synthèses proposées sont difficilement applicables à l'échelle industrielle, impliquant notamment l'utilisation de métaux alcalins très réactifs vis-à-vis de l'eau et de l'oxygène de l'air.

Ainsi, le problème technique que se propose de résoudre la Demanderesse par la présente invention est la mise à disposition de nouvelles molécules à fort pouvoir odorant, stables, ayant une durée de conservation longue, et préparées selon un mode de réalisation simple et à partir de matières premières peu onéreuses et abondantes.

La simplicité de mise en oeuvre des réactions et le faible coût des matières premières utilisées, représentent des avantages importants pour la mise en oeuvre industrielle de l'invention.

La présente invention a pour objet l'utilisation de la 5,5-diméthyl-3-éthyl-3,4-dihydro-furan-2-one (ou Noxolide) comme agent ou composé odorant, notamment en tant que fragrance ou arôme. Un panel d'évaluation, composé de plusieurs professionnels, a évalué qualitativement le composé de l'invention. La Noxolide, représentée ci-dessous par la formule (A), a été décrite comme marine iodée avec des facettes noix, anis un peu réglisse, patchouli, damascénone.

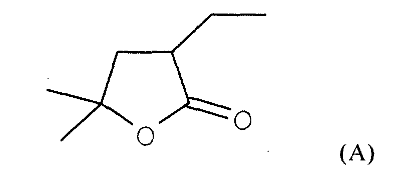

Du fait de ses qualités olfactives, la Noxolide trouve un emploi très varié en parfumerie pour la préparation de compositions parfumantes, de bases ou concentrés parfumants, parfums ou eaux de toilette, ainsi que dans la préparation d'articles parfumés divers, tels que savons, bain moussant, gels douches ou de bain, lotions après rasage, shampooings ou autres produits capillaires notamment d'hygiène capillaire ou de soin capillaire, préparations cosmétiques, désodorisants corporels ou d'air ambiant, ou encore détergents ou adoucissants textiles ou produits d'entretien ou d'assainissement de l'air ambiant.

En particulier, l'invention a pour objet un produit d'hygiène ou de soin capillaire, **caractérisé en ce qu**'il comprend de la 5,5-diméthyl-3-éthyl-3,4-dihydro-furan-2-one.

Dans ces applications, le composé selon l'invention peut être employé seul ou, comme il est plus courant en parfumerie, en mélange avec d'autres ingrédients parfumants, des solvants ou des adjuvants d'usage courant en parfumerie et que l'homme du métier est à même de choisir en fonction de l'effet désiré et de la nature du produit à parfumer.

La présente invention a également pour objet l'utilisation de la 5,5-diméthyl-3-éthyl-3,4-dihydro-furan-2-one (Noxolide) en tant qu'arôme. La Noxolide est utile comme arôme alimentaire, particulièrement pour la préparation de compositions alimentaires, d'additifs alimentaires, de bases et concentrés aromatisants ainsi que dans la préparation de produits aromatisés divers, tels que boissons, produits laitiers, crèmes glacées, soupes, sauces, plats cuisinés, produits à base de viande, aides culinaires, biscuits ou snacks salés. La Noxolide peut également être utilisée en tant qu'arôme dans des vins, bières et/ou tabacs.

La présente invention a également pour objet l'utilisation de la 5,5-diméthyl-3-éthyl-3,4-dihydro-furan-2-one (Noxolide) en tant qu'agent masquant d'un goût et/ou d'une odeur, notamment dans une composition alimentaire, ou cosmétique, ou pharmaceutique.

Dans ces applications, le composé selon l'invention peut être employé seul ou, comme il est plus courant en aromatique, en mélange avec d'autres ingrédients aromatisants, des solvants ou des adjuvants d'usage courant en aromatique alimentaire et que l'homme du métier est à même de choisir en fonction de l'effet désiré et de la nature du produit à aromatiser.

Les concentrations dans lesquelles la 5,5-diméthyl-3-éthyl-3,4-dihydro-furan-2-one (Noxolide) peut être utilisée pour obtenir les effets parfumant, aromatisant et/ou masquant désirés varient dans une gamme de valeur très étendue, de 0,001% à 99% en poids, de préférence de 0,001% à 20% en poids, très préférentiellement de 0,1% à 10% en poids, étant bien connu que ces valeurs dépendent de la nature de l'article à parfumer, de l'effet odorant recherché, et de la nature des autres ingrédients dans une composition donnée.

L'invention a également pour objet un nouveau procédé de synthèse de la 5,5-diméthyl-3-éthyl-3,4-dihydro-furan-2-one, (Noxolide) comprenant
- une première étape [1] faisant réagir du méthylate de sodium et du malonate de diméthyle avec du 3-chloro-2-méthylprop-1-ène, aboutissant à la formation du 4-méthyl-2-méthoxycarbcnyl-pent-4-éncate de méthyle (I),
- une deuxième étape [2] faisant réagir le produit (I) avec du t-butylate de potassium et du bromure d'éthyle, aboutissant à la formation du 2-éthyl-4-méthyl-2-méthoxycarbonyl-pent-4-énoate de méthyle (II),
- une troisième étape [3] faisant réagir le produit (II) avec de la soude et de l'acide phosphorique, aboutissant à la formation de 5,5-diméthyl-3-éthyl-3,4-dihydro-furan-2-one (A).

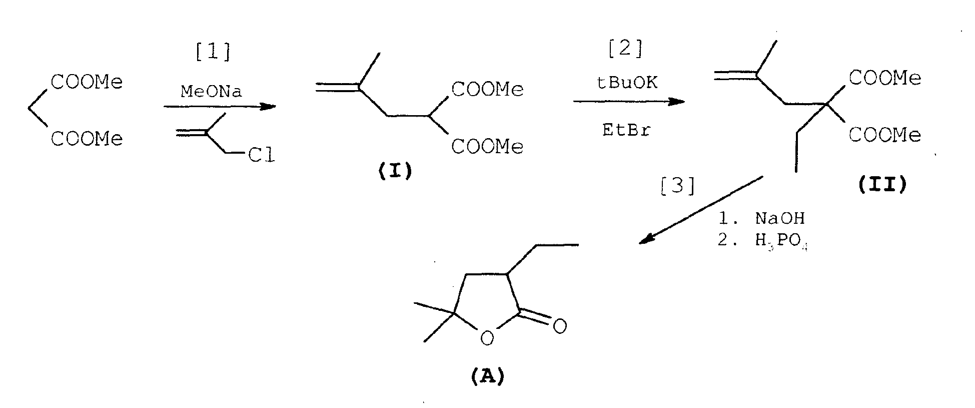

Les exemples suivants illustrent davantage les différents procédés de fabrication des composés nouveaux selon l'invention ainsi que leur utilisation, et leur intérêt. Ces exemples ne sont présentés que dans un but d'illustration et ne peuvent être considérés comme limitatifs de l'invention.

### Exemple 1 : Production de la Noxolide ou 5,5-diméthyl-3-éthyl-3,4-dihydro-furan-2-one

### 4-méthyl-2-méthoxycarbonyl-pent-4-ènoate de méthyle (I)

La réaction est conduite dans un ballon tricol de 1L muni d'un thermomètre plongeant, d'un réfrigérant ascendant équipé d'une garde séchante à potasse et d'une ampoule de coulée isobare de 250mL.

Dans le ballon sec et purgé à l'azote, sont introduits successivement 54g de méthylate de sodium, 300mL de méthanol anhydre, 1g d'iodure de potassium puis 138,7g de malonate de diméthyle. La solution résultante est agitée une heure à température ambiante puis 95g de chloro-3 méthyl-2 propène-1 sont ajoutés lentement de l'ampoule de coulée. Le mélange est agité 3 heures à température ambiante puis 12 heures au reflux. Le réfrigérant est remplacé par un ensemble à distillation et le solvant est distillé. Le résidu est repris par 50mL d'eau claire et 300mL de méthylcyclohexane. La phase organique est séchée puis le solvant est éliminé par distillation. Le résidu est fractionné à l'aide d'une colonne Vigreaux de 30cm ; 3 fractions sont collectées :
- La première fraction, qui pèse 15g, est constituée de malonate de diméthyle (Eb = 85-87°C/20mm Hg)
- La seconde fraction pèse 138g et bout à 86°C/6mm Hg. Elle est constituée de l'ester recherché, (I), soit un rendement de 74% (80% par rapport au produit de départ effectivement consommé). Le produit est pur en Chromatographie en Phase Vapeur à plus de 98%.
- Une troisième fraction est obtenue par distillation sous haut vide (Eb = 64-68°C/1mm Hg). Elle pèse 36g et a été identifié comme le bis(2-methyl-2-propènyl)-2-malonate de diméthyle.

### Pour le composé I :

IR : (NaCl, film) 1760, 1740,1440, 1350, 1295, 1230, 1150, 1050, 1020, 900 cm⁻¹
RMN ¹H , (CDCl₃ )
1.73 ppm (m ; 3H) ; 2.50 (d ;J = 8Hz ;2H) ; 3.20 à 3.70 (m ; ;H) ; 3.68 (s ; 6H) ; 4.72(m 2H) .
MS : 70 eV
   186 (M^{+.}) ; 154 ; 127 ; 111 ; 95 uma

### 2-éthyl-4-méthyl-2-méthoxycarbonyl-pent-4-ènoate de méthyle (II)

La réaction est conduite dans un ballon tricol de 1L muni d'un thermomètre plongeant, d'un réfrigérant ascendant équipé d'une garde séchante à potasse et d'une ampoule de coulée isobare de 250mL.

Dans le ballon sont introduits successivement 93g de 4-Methyl-2-méthoxycarbonyl-pent-4-ènoate de méthyle (I) et 40g de DMSO anhydre. Le mélange étant maintenu entre 20 et 30°C à l'aide d'un bain réfrigérant, 59g de t-butylate de potassium sont ajoutés puis le mélange obtenu est agité 2 heures à température ambiante avant que 35,5g de bromure d'éthyle soient additionnés lentement dans le ballon par l'intermédiaire de l'ampoule de coulée de telle sorte que la température ne dépasse pas 30°C dans le milieu. La solution obtenue est agitée 12 heures à température ambiante puis versée sur 300 mL d'acide chlorhydrique à 5%. La phase aqueuse résultante est extraite 5 fois avec 100 ml de méthylcyclohexane. Les phases organiques réunies sont lavées avec 100mL d'eau claire puis séchées et concentrées sous vide. Le résidu est rectifié sous vide. Le produit distille à 95 °C/4 mm Hg.
91 g de produit recherché sont isolés soit un rendement de 85%.
RMN ¹H , (CDCl₃)
   0.64 ppm (t, 7.5 Hz, 3H) ; 1.06 (t, 7.1 Hz, 6H) ; 1.46 (s, 3H) ,1.75 (q, 7.5 HZ, 2H) ; 2.50 (s, 2H) ;4.00 (q, 7.1 Hz, 4H) ; 4.53 (s, 1H) ; 4.65 (s, 1H) .
IR : (NaCl, film) 3079, 2955, 1736, 1647, 1437, 1301, 1225, 1123, 901 cm ⁻¹
MS : 214 (M^{+.} ) ; 185 ; 155 ; 139 ; 123 ; 95 ; 85 ; 79 ; 67 uma

### 5,5-diméthyl-3-éthyl-3,4-dihydro-furan-2-one

La réaction est conduite dans un ballon tricol de 1L muni d'un thermomètre plongeant et d'un réfrigérant ascendant. Ce dernier sera ensuite remplacé par un système de distillation classique : colonne Vigreaux, condenseur et recette.

Dans le ballon sont introduits successivement 200mL d'eau claire, 200mL de méthanol, 20g de soude en paillettes puis 53,5g de 2-éthyl-4-méthyl-2-méthoxycarbonyl-pent-4-ènoate de méthyle (II). Le mélange est porté 4 heures au reflux puis le chauffage est interrompu. Le montage à distiller remplace le réfrigérant ascendant et le solvant est distillé jusqu'à obtenir 90°C dans les vapeurs. De l'eau chaude est introduite, au fur et à mesure, dans le ballon afin de conserver une fluidité au mélange en distillation. La phase aqueuse est portée à 60°C, elle est extraite avec 2 fois 100 mL de toluène puis elle est portée entre 0 et 5°C. Un lourd précipité blanc se forme. La suspension est acidifiée jusqu'à pH=3 avec une solution d'acide sulfurique 6N, tout en maintenant la température inférieure à 5°C. Le précité blanc est filtré, lavé à l'eau claire jusqu'à obtenir la neutralité puis il est repris dans 300mL de toluène sec et porté au reflux en présence d'lg d'acide phosphorique à 85% pendant 12 heures.

La solution est refroidie, lavée à neutralité avec une solution saturée de bicarbonate de sodium puis séchée. Le solvant est distillé sous vide partiel puis le résidu est rectifié sous vide (Eb=86°C/6 mm Hg ). La lactone pèse 27.8g, ce qui représente un rendement de 78,2%.

Elle possède une forte odeur de noix verte. Elle est nommée Noxolide (Formule A).
RMN ¹H , (CDCl₃ )
   1.0 ppm (t, 7.4 Hz, 3H) ; 1.4(s, 3H) ; 1.5(s, 3H) ; 1.4 à 1.6(m, 1H) ; 1.7(m, 1H) ; 1.8 à 2.0(m, 1H) ; 2.3(dd , 12.6/ 8.9 Hz, 1H) ; 2.7(ddt, 11.2/8.9/4.7 Hz, 1H)
IR : (NaCl, film) 2973, 2935, 2878, 1768, 1461, 1376, 1271, 1168, 1118, 952 cm⁻¹
MS : (70 eV) 142 (M+.) ; 127 ; 99 ; 83 ; 69 uma
RMN ¹³C : CDCl₃ , 50MHz
   12.0, 24.1, 27.5, 29.3, 41.0, 42.4, 82.5, 178.5 ppm

### Analyses Elémentaires :

| | | | | |
|---|---|---|---|---|
| % calc. | C | 67.57 | H | 9.92 |
| % obt. | C | 66.84 | H | 10.02 |

### Constantes Physiques :

N₂₀ = 1.4355
D₂₀ = 0.949

### Exemple 2 : Evaluation olfactive de la Noxolide dans une composition

La Noxolide, a été décrite olfactivement comme marine iodée avec des facettes noix, anis un peu réglisse, patchouli, damascénone.

Une composition parfumante a été crée (essai 2) dans laquelle l'impact olfactif de la noxolide a été examinée par comparaison avec une composition ne contenant pas le composé (essai 1).

| **Composants** | **Essai 1** | **Essai 2** |
|---|---|---|
| | (poids) | (poids) |
| HS MELON P-99 | 10,00 | 10,00 |
| BERGAMOTTE 77 P-99 | 15,00 | 15,00 |
| LINALYLE ACETATE HLR | 50,00 | 50,00 |
| CASSIS BASE 345 B | 5,00 | 5,00 |
| CITRON TERPENES USA | 50,00 | 50,00 |
| DIHYDROMYRCENOL | 50,00 | 50,00 |
| ETHYL LINALOL (Givaudan) | 90,00 | 90,00 |
| FLORALOZONE (IFF) | 10,00 | 10,00 |
| GALAXOLIDE sans PE dans MIP | 135,00 | 135,00 |
| METHYL DIHYDRO JASMONATE | 200,00 | 200,00 |
| OCTAHYDRO TETRAMETHYL ACETCNAPHTCNE | 40,00 | 40,00 |
| LILIAL (Givaudan) du MFI | 100,00 | 100,00 |
| LINALOL HLR | 50,00 | 50,00 |
| GAMMA NONALACTONE-PRUNOLIDE | 50,00 | 50,00 |
| ALDEHYDE C14 GAMMA UNDECA (10% DPG) | 5,00 | 5,00 |
| DIHYDROFLORIFONNE (IFF) (10% DPG) | 5,00 | 5,00 |
| FOLIONE (Givaudan) (1% DPG) | 20,00 | 20,00 |
| JASMOLACTONE (Firmenich) (10% DPG) | 5,00 | 5,00 |
| TRIPLAL (IFF) (10% DPG) | 5,00 | 5,00 |
| VANILLINE (Rhone-Poulenc) (10% DPG) | 5,00 | 5,00 |
| DPG | 2,70 | 0,00 |
| NCXOLIDE | 0,00 | 2,70 |

Les compositions précédentes ont été testées dans une application shampooing de type 2 en 1 à raison de 0.5% dans la base.

La formule de l'essai 2 donne plus de puissance et d'accroche en tête. L'aspect plus vert et plus naturel que la formule de l'essai 1 est remarquable. La Noxolide améliore particulièrement le côté vert aqueux naturel transparent de l'accord parfum. Elle souligne les tonalités hespéridées pétillantes ainsi que l'accord lactonique, fruité. La formule de l'essai 1 est douce, musquée, plate et sans relief. Elle masque difficilement la base.

## Revendications

1. Utilisation de la 5,5-diméthyl-3-éthyl-3,4-dihydro-furan-2-one, en tant qu'agent ou composé odorant, notamment en tant que fragrance ou arôme.

2. Utilisation de la 5,5-diméthyl-3-éthyl-3,4-dihydro-furan-2-one selon la revendication 1, en tant qu'arôme alimentaire, ou en tant qu'arôme dans le vin, la bière ou le tabac.

3. Utilisation de la 5,5-diméthyl-3-éthyl-3,4-dihydro-furan-2-one selon la revendication 1, pour la préparation de compositions alimentaires, d'additifs alimentaires, de bases ou concentrés aromatisants.

4. Utilisation de la 5,5-diméthyl-3-éthyl-3,4-dihydro-furan-2-one selon la revendication **3,** dans laquelle ladite composition alimentaire est choisi dans le groupe comprenant les boissons, produits laitiers, crèmes glacées, soupes, sauces, plats cuisinés, produits à base de viande, aides culinaires, biscuits ou snacks salés.

5. Utilisation de la 5,5-diméthyl-3-éthyl-3,4-dihydro-furan-2-one selon la revendication 1, pour la préparation de compositions parfumantes, de bases ou concentrés parfumants, parfums ou eaux de toilette, savons, bain moussant, gels douches ou de bain, lotions après rasage, préparations cosmétiques, désodorisants corporels ou d'air ambiant, ou encore détergents ou adoucissants textiles, produits d'entretien et d'assainissement de l'air ambiant.

6. Utilisation de la 5,5-diméthyl-3-éthyl-3,4-dihydro-furan-2-one selon la revendication **1,** en tant qu'agent masquant d'un goût ou d'une odeur.

7. Utilisation selon l'une quelconque des revendications **1 à 6,** dans laquelle la 5,5-diméthyl-3-éthyl-3,4-dihydro-furan-2-one est en combinaison avec au moins un autre ingrédient aromatisant ou parfumant, et/ou au moins un solvant, et/ou au moins un adjuvant.

8. Utilisation selon l'une quelconque des revendications **1 à 7,** dans laquelle la 5,5-diméthyl-3-éthyl-3,4-dihydro-furan-2-one est présente dans une concentration égale à 0,001% à 99% en poids, de préférence de 0,001% à 20% en poids, très préférentiellement 0,1% à 10% en poids, par rapport au poids total.

9. Procédé de synthèse de la 5,5-diméthyl-3-éthyl-3,4-dihydro-furan-2-one comprenant
- de faire réagir du méthylate de sodium et du malonate de diméthyle avec du 3-cholro-2-méthylprop-1-ène,
- de faire réagir le produit issu de la première étape ci-dessus avec du t-butylate de potassium et du bromure d'éthyle,
- de faire réagir le produit issu de la seconde étape ci-dessus avec de la soude et de l'acide phosphorique.

10. Produit d'hygiène ou de soin capillaires, **caractérisé en ce qu'**il comprend de la 5,5-diméthyl-3-éthyl-3,4-dihydro-furan-2-one.

## Claims

1. Use of 5,5-dimethyl-3-ethyl-3,4-dihydrofuran-2-one as odorous agent or compound, in particular as fragrance or flavor.

2. The use of 5,5-dimethyl-3-ethyl-3,4-dihydrofuran-2-one according to claim 1 as food flavor or as flavor in wine, beer or tobacco.

3. The use of 5,5-dimethyl-3-ethyl-3,4-dihydrofuran-2-one according to claim 1 in the preparation of food compositions, food additives, flavoring bases or concentrates.

4. The use of 5,5-dimethyl-3-ethyl-3,4-dihydrofuran-2-one according to claim 3, in which said food composition is chosen from the group comprising drinks, dairy products, ice creams, soups, sauces, ready-made meals, meat-based products, cooking aids, biscuits and salty snacks.

5. The use of 5,5-dimethyl-3-ethyl-3,4-dihydrofuran-2-one according to claim 1 in the preparation of scenting compositions, scenting bases or concentrates, perfumes or eaux de toilette, soaps, foam bath, shower or bath gels, aftershave lotions, cosmetic preparations, deodorants for the body or for the surrounding air, or also textile detergents or softeners or products for the upkeep of or for purifying the surrounding air.

6. The use of 5,5-dimethyl-3-ethyl-3,4-dihydrofuran-2-one according to claim 1 as masking agent for a taste or a smell.

7. The use according to any one of claims 1 to 6, in which 5,5-dimethyl-3-ethyl-3,4-dihydrofuran-2-one is in combination with at least one other flavoring or scenting ingredient and/or at least one solvent and/or at least one adjuvant.

8. The use according to any one of claims 1 to 7, in which 5,5-dimethyl-3-ethyl-3,4-dihydrofuran-2-one is present in a concentration of from 0.001% to 99% by weight, preferably from 0.001% to 20% by weight, very preferably from 0.1% to 10% by weight, with respect to the total weight.

9. A process for the synthesis of 5,5-dimethyl-3-ethyl-3,4-dihydrofuran-2-one, comprising
- reacting sodium methoxide and dimethylmalonate with 3-chloro-2-methylprop-1-ene,
- reacting the product resulting from the above first stage with potassium t-butoxide and ethyl bromide,
- reacting the product resulting from the above second stage with sodium hydroxide and phosphoric acid.

10. A hair hygiene or care product, **characterized in that** it comprises 5,5-dimethyl-3-ethyl-3,4-dihydro-furan-2-one.

## Patentansprüche

1. Verwendung von 5,5-Dimethyl-3-ethyl-3,4-dihydrofuran-2-on als Duftmittel oder -verbindung, insbesondere als Parfüm oder Aroma.

2. Verwendung von 5,5-Dimethyl-3-ethyl-3,4-dihydrofuran-2-on nach Anspruch 1 als Lebensmittelaroma oder als Aroma in Wein, Bier oder Tabak.

3. Verwendung von 5,5-Dimethyl-3-ethyl-3,4-dihydrofuran-2-on nach Anspruch 1 zur Herstellung von Lebensmittelzusammensetzungen, Lebensmittelzusatzstoffen, Aromatisierungsbasen oder -konzentraten.

4. Verwendung von 5,5-Dimethyl-3-ethyl-3,4-dihydrofuran-2-on nach Anspruch 3, wobei die Lebensmittelzusammensetzung aus der Gruppe ausgewählt ist, umfassend Getränke, Milchprodukte, Eiscremes, Suppen, Soßen, Fertiggerichte, Produkte auf Fleischbasis, Kochhilfsmittel, Backwerk oder gesalzene Snacks.

5. Verwendung von 5,5-Dimethyl-3-ethyl-3,4-dihydrofuran-2-on nach Anspruch 1 zur Herstellung von Parfümierungszusammensetzungen, Parfümierungsbasen oder -konzentraten, Parfüms oder Eaux de Toilette, Seifen, Schaumbad, Dusch- oder Badegelen, Aftershavelotionen, Kosmetikzubereitungen, Körper- oder Umgebungsluftdeodorants oder auch Detergenzien oder Textilweichspülern, Produkten zur Pflege oder Reinigung der Umgebungsluft.

6. Verwendung von 5,5-Dimethyl-3-ethyl-3,4-dihydrofuran-2-on nach Anspruch 1 als Mittel zum Überdecken eines Geschmacks oder Geruchs.

7. Verwendung nach einem der Ansprüche 1 bis 6, wobei 5,5-Dimethyl-3-ethyl-3,4-dihydrofuran-2-on mit mindestens einem anderen Aromatisierungs- oder Parfümierungsinhaltsstoff und/oder mindestens einem Lösungsmittel und/oder mindestens einem Zusatzstoff kombiniert ist.

8. Verwendung nach einem der Ansprüche 1 bis 7, wobei 5,5-Dimethyl-3-ethyl-3,4-dihydrofuran-2-on in einer Konzentration von 0,001 bis 99 Gew.-%, vorzugsweise von 0,001 bis 20 Gew.-%, ganz besonders von 0,1 bis 10 Gew.-%, bezogen auf das Gesamtgewicht vorliegt.

9. Verfahren zur Synthese von 5,5-Dimethyl-3-ethyl-3,4-dihydrofuran-2-on, bei dem man
- Natriummethylat und Dimethylmalonat mit 3-Chlor-2-methylprop-1- en umsetzt,
- das aus dem vorstehenden ersten Schritt erhaltene Produkt mit Kalium-t-butylat und Ethylbromid umsetzt,
- das aus dem vorstehenden zweiten Schritt erhaltene Produkt mit Natronlauge und Phosphorsäure umsetzt.

10. Haarhygiene- oder Haarpflegeprodukt, **dadurch gekennzeichnet, dass** es 5,5-Dimethyl-3-ethyl-3,4-dihydrofuran-2-on umfasst.
